# EUROPEAN PATENT SPECIFICATION

(11) **EP 1 171 046 B1**
(45) Date of publication and mention of the grant of the patent: **14.07.2004**
(21) Application number: 99933063.2
(22) Date of filing: 27.07.1999
(51) Int. Cl.: A61B 17/64

(54) **BONE FIXING DEVICE**
KNOCHENFIXATIONSVORRICHTUNG
DISPOSITIF DE FIXATION D'OS

(30) Priority: 08.04.1999 ZA 9902585
(43) Date of publication of application: 16.01.2002
(73) Proprietor: Röthig, Thomas, Max, 7441 Table View (ZA)
(72) Inventor: RÖTHIG, Hermann, Florian, 7530 Welgemoed (ZA)
(74) Representative: Harrison Goddard Foote
(86) International application number: PCT/IB1999/001325
(87) International publication number: WO 2000/061019

(56) References cited:
- EP-A- 0 153 546
- WO-A-91/11149
- CH-A- 303 453
- DE-A- 3 805 178
- FR-A- 2 557 933
- US-A- 4 483 334
- US-A- 5 624 440
- R.TEXHAMMAR AND C.C.COLTON (EDS.): "AO/ASIF Instruments and Implants" 1994 , SPRINGER-VERLAG , BERLIN & HEIDELBERG XP002125358 236780 page 321 -page 325

## Description

Bone fixing devices are used for various orthopaedic purposes, and many different types of such devices are known.

Texhammer and Coulton: "AO/ASIF Instruments and Implants" 1994, Springer-Verlag disclose an external fixator tubular system having a number of tubes and screws, joints for joining adjacent tubes, and clamps for gripping implant screws.

An articulation device for the assembly of several elements by means of linkage members is described in FR2557933. Elements to be assembled and linkage members between these elements each comprise at least one annular notched face which is capable of being engaged with a corresponding toothed annular face of another element or linkage member, the said toothed annular face having a central bore for the passage of a member for fixing one element to a linkage member. The device is used for the assembly of elements especially for an external fixture intended for osteosynthesis.

US 4483334 discloses an external fixation device for holding bone segments in known relation to each other, which includes a pair of bone clamp assemblies each secured to bone pins extending from the bone segments, a bridge extending between the pin clamp assemblies and a specialized high friction universal assembly connecting the bridge to each of the pin clamp assemblies.

A compact external fixator used to repair small bone fractures is shown in US 5624440, which includes an elongate cylindrical support rod to which are mounted a pair of articulation assemblies. Each assembly is held to the support rod by a rod clamp which, in a loosened condition, can rotate about and translate along the support rod. When tightened, the clamps fix the positions of the articulation assemblies relative to the rod. A pin carrier block is pivotally attached to each rod clamp to pivot about a first pivot axis that is perpendicular to and offset from the rod. As with the clamp, the pivotal motion of the block can be selectively fixed or loosened. Each block further includes a generally planar circular pin mounting shelf with a central axis normal to the plane of the shelf. The plane of the shelf is substantially parallel to and offset from the first pivot axis. A generally planar and disk-like pin clamp ring is disposed centered coaxially over the central axis of each pin shelf. Each ring includes a first face in which are formed a pair of spaced apart, parallel pin guide tracks, with the first face being disposed toward and spaced apart from the pin shelf. A pair of pins is captured between the first face and the pin shelf, with the pins located in the pin guide tracks. The pins and pin clamp rings are rotatable about the central axis of the pin shelf until the ring is tightened.

The known devices generally are provided with various types of pin clamping devices. In most cases these are not adjustable in various directions, and such adjustability is desirous in many instances.

It is an object of the invention to provide a bone fixing device having pin clamping units which are adjustable in various directions.

### SUMMARY OF INVENTION

According to the invention, there is provided a bone fixing device, which includes at least one elongated link, and a number of clamp units movably mounted on one such elongated link, each elongated link being substantially round in end view, and each clamp unit including a clamp body slidably and rotatably mounted on its link, with clamp locking means being provided for locking the clamp body on the link, the clamp body further rotatably carrying a bone pin carrier, which is adapted to receive at least one bone pin, the bone pin carrier including a base disc rotatably mounted on the clamp body, and a top disc, carrier locking means being provided for locking the bone pin carrier relative to the clamp body, the carrier locking means being adapted to pull the top disc and the base disc relatively together to clamp at least one bone pin between them; the top disc and the base disc have cooperating locating grooves for receiving bone pins; characterised in that a pin is provided in the base disc extending towards the top disc and a hole is provided in the top disc for receiving the pin so as to prevent relative rotation between the base disc and the top disc for aligning the locating grooves of the top disc and the base disc for receiving and locating the bone pin(s).

The elongated link may be an elongated cylindrical bar.

A number of elongated links may be provided, the links being connected in series by link connecting means.

The link connecting means may include a projection at an end of a link, and a locking surface provided on the projection adapted to be locked to a similar locking surface provided on a projection of another link.

The locking surface may be a ratchet surface.

The clamp body may include a hole for receiving its link and a split gap extending outwardly from the hole, and the clamp locking means being adapted to pull the body together at its split gap to clamp the body onto its link.

The clamp locking means may include a hole in the clamp body extending transversely through the split gap, and a bolt adapted to be passed through the hole for pulling the body together.

The carrier locking means may include a threaded bolt adapted to pass through a hole in the top disc and a hole in the base disc to be threaded into a threaded hole in the clamp body.

A connecting link with at least one groove may be provided, and link connectors for connecting the connecting link to two elongated links.

At least one of the elongated links may include an outer sleeve and an inner rod extending out of the outer sleeve and being connected to another elongated link, and adjustment means for adjusting the inner rod relative to the outer sleeve.

### BRIEF DESCRIPTION OF DRAWINGS

The invention will now be described by way of example with reference to the accompanying schematic drawings.

In the drawings there is shown in:
- Figure 1: a side view of a first embodiment of a bone fixing device in accordance with the invention;
- Figure 2: on an enlarged scale, a side view of one link of the device illustrated in Figure 1;
- Figure 3: a view seen along arrow III in figure 2;
- Figure 4: an end view seen along arrow IV in Figure 2;
- Figure 5: on an enlarged scale, a view of a second link of the device illustrated in Figure 1;
- Figure 6: an end view seen along arrow VI in Figure 5;
- Figure 7: a view seen along arrow VII in Figure 5;
- Figure 8: on an enlarged scale, an end view of the base part of a bone clamping unit seen along arrow VIII in Figure 1;
- Figure 9: a view seen along arrow IX in Figure 8;
- Figure 10: a plan view seen along arrow X in Figure 8;
- Figure 11: a view of a first bolt for use in the unit illustrated in Figures 8 to 10;
- Figure 12: a view of a second bolt for use in the device illustrated in Figures 8 to 10;
- Figure 13: on an enlarged scale, a plan view of the base disc of the bone clamping unit illustrated in Figure 1;
- Figure 14: a side view seen along XIV in Figure 13;
- Figure 15: a plan view seen along arrow XV in Figure 13;
- Figure 16: on an enlarged scale, a plan view of the top disc of a bone clamping unit as illustrated in Figure 1;
- Figure 17: a view seen along arrow XVII in Figure 16;
- Figure 18: a view seen along arrow XVIII in Figure 16;
- Figure 19: on an enlarged scale, a side view of another link for use in the device illustrated in Figure 1;
- Figure 20: an end view seen along arrow XX in Figure 19;
- Figure 21: an end view seen along arrow XXI in Figure 19;
- Figure 22: on an enlarged scale, a side view of a further type of link for use in the bone fixing device illustrated in Figure 1;
- Figure 23: an end view seen along arrow XXIII in Figure 22;
- Figure 24: a view seen along arrow XXIV in Figure 22;
- Figure 25: on a reduced scale, a plan view of a pelvic bone fixing device including various components as illustrated in the preceding drawings;
- Figure 26: a view seen along arrow XXVI in Figure 25;
- Figure 27: on an enlarged scale, a view of the connecting link in the pelvic bone fixing device illustrated in Figure 25;
- Figure 28: an end view seen along arrow XXVIII in Figure 27;
- Figure 29: on an enlarged scale, an exploded view of the link connector shown in Figures 25 and 26;
- Figure 30: on an enlarged scale, a view of the end pin holder illustrated in Figures 25 and 26;
- Figure 31: a view seen along arrow XXXI in Figure 30;
- Figure 32: a view seen along arrow XXXII in Figure 30;
- Figure 33: on an enlarged scale, a view of the base disc of the end pin holder illustrated in Figures 25 and 26;
- Figure 34: a view seen along arrow XXXIV in Figure 33;
- Figure 35: a view seen along arrow XXXV in Figure 33;
- Figure 36: on an enlarged scale, a view of the outer disc of the pin holder illustrated in Figures 25 and 26;
- Figure 37: a view seen along arrow XXXVII in Figure 36;
- Figure 38: a view seen along arrow XXXVIII in Figure 36; and
- Figure 39: an exploded view of an adjustable link for use with the bone fixing device illustrated in Figure 1.

### DETAILED DESCRIPTION OF DRAWINGS

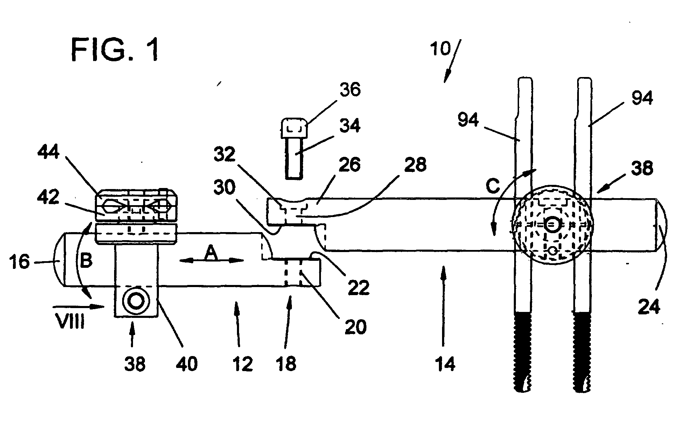

Referring to Figure 1, an orthopaedic or bone fixing device in accordance with the invention, generally indicated by reference numeral 10, includes a first link 12 and a second link 14. The links 12, 14 are made of round solid metal bars but also can be made of hollow cylindrical material if required.

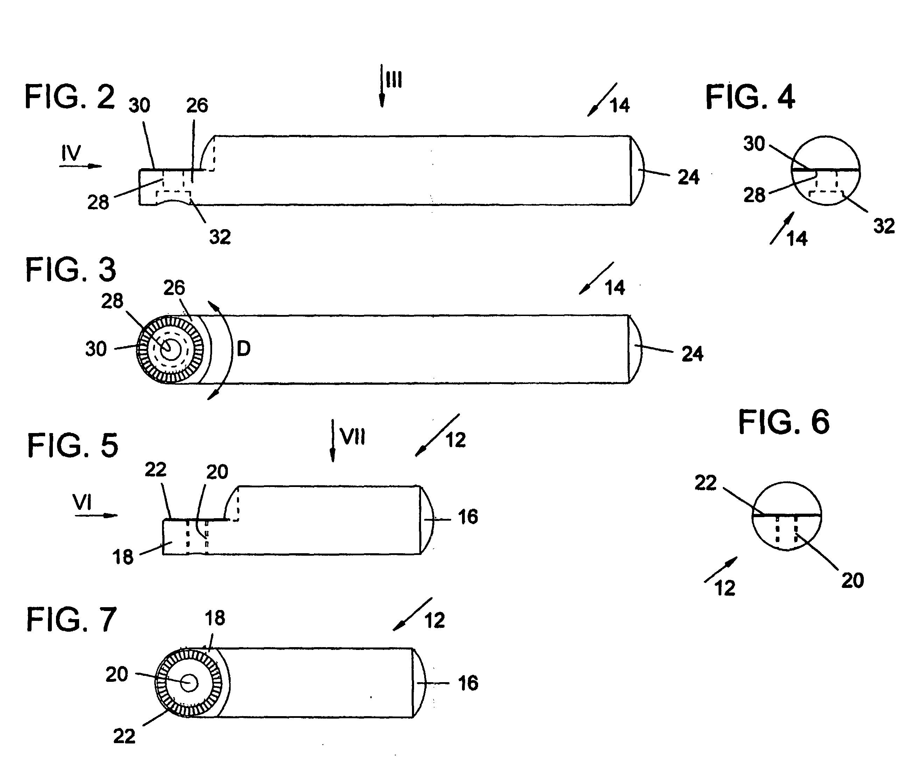

The first link 12 (also illustrated in Figures 5 to 7) has a rounded end 16 and, opposite thereto, a projecting connector part 18 having an internally threaded hole 20 and a circular ratchet surface 22.

The second link 14 has a rounded end 24 and, opposite thereto, a converting part 26 having a hole 28 and a circular ratchet surface 30.

The hole 28 is recessed at 32 as shown.

The second link 14 is also illustrated in Figures 2 to 4.

In use the links 12, 14 are placed together so that their ratchet surfaces 22, 30 are in engagement. The links 12, 14 are rotated to be in a required position, either being in alignment or defining any angle as desired between them. Then the threaded bolt 34 is screwed into the threaded hole 20 until its head 36 is received in the recess 32. The head 36 is a so-called Allan-screw.

The links 12, 14 carry bone pin holders 38.

The pin holders 38 include a link connector body 40, a rotatable base disc 42 and an upper disc 44.

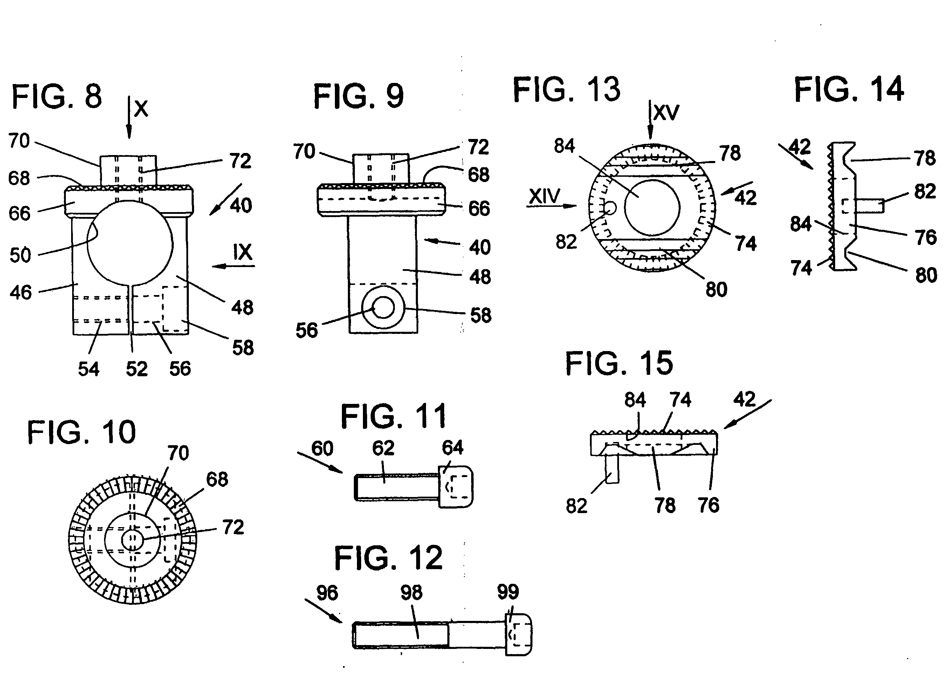

Details of the base part are illustrated in Figures 8 to 10. The body 40 includes a cylindrical body which is split into two body parts 46, 48 defining a circular hole 50 therein by way of a gap or passage 52 extending to the outside. An internally threaded hole 54 is provided in the part 46 and a hole 56 with a recess 58 in the part 48. The screw threaded bolt 60 shown in Figure 11 having a threaded shank 62 and a head 64 is passed through the passage 56 and screwed into the threaded hole 54 for clamping the parts 46, 48 onto a link 12, 14 received in the hole 50.

The body 40 has a disc shaped head 66 of which the upper face 68 has a circular ratchet shape. A pillar or boss 70 extends outwardly from the head 66 and a threaded passage 72 is provided in the boss 70.

The base disc 42, which is also illustrated in Figures 13 to 15, includes a bottom ratchet shaped surface 74 and opposite thereto an engagement surface 76 in which two parallel grooves 78, 80 which are truncated shaped in end view, are provided. A pin 82 extends from the surface 78. The disc 42 has a central hole 84 for receiving the boss 70. (If required the faces 68, 74 need not be ratchet shaped but only be in frictional contact for locking together).

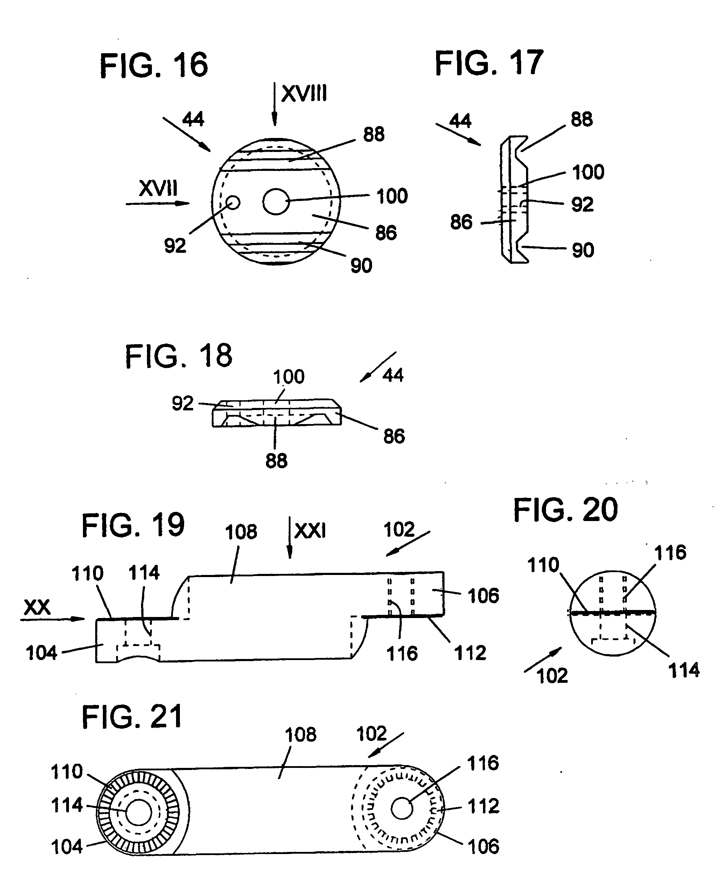

The upper disc 44, also illustrated in Figures 16 to 18, includes a bottom surface 86 in which two recesses 88, 90 which are of truncated shape in end view, are provided. The disc 44 has a hole 92 for receiving the pin 82 of the disc 42 so as to align the recesses 78, 80 and 88, 90 for receiving bone 94 as shown in Figure 1.

A screw or bolt 96, illustrated in Figure 12, has a threaded shank 98 and a head 99. It is passed through the hole 100 in the disc 44 and is screwed into the threaded hole 72 in the boss 70 of the body 40.

The disc 42 and 44 can be rotated as required into any angular position and then are fixed in this position by way of the bolt 96.

As is shown in Figure 1 the bone pin holder 38 can be moved longitudinally on its link 12 or 14 as indicated by arrow A, or be rotated on its link 12 or 14 as indicated by arrow B, and the base disc 42 and top disc 44 can be rotated as indicated by arrow C.

The bone pins 44 therefore can be moved in three directions making numerous angles and positions possible.

The links 12, 14 can be pivotted relative to each other as indicated by arrow D in Figure 3. This applies to all other ratchet contact surfaces of components shown in the drawings.

In Figures 19 to 21 a further embodiment of a link is shown.

This link, generally indicated by reference numeral 102, has two opposite connector parts 104, 106 which are on opposite sides of the longitudinal central axis of the cylindrical body 108. They have circular ratchet surfaces 110 and 112 respectively and a hole 114 and an internally threaded hole 116 respectively. These ratchet surfaces 110 and 112 are fitted to complementary ratchet surfaces of further links as required.

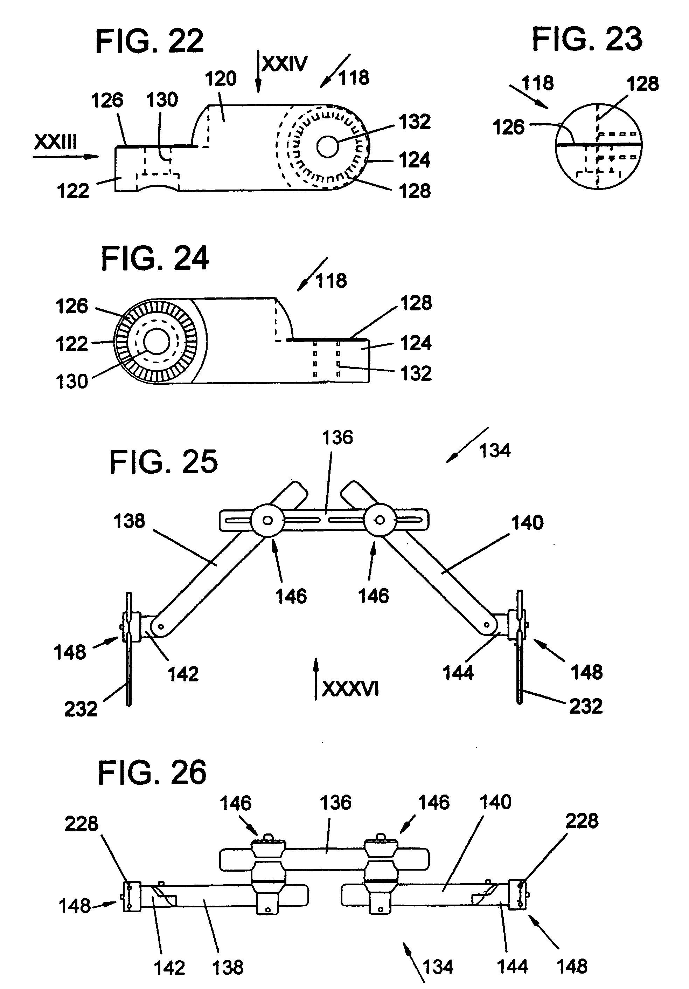

In Figures 22 to 24 a further embodiment of a link is shown. This link, generally indicated by reference numeral 118, includes a cylindrical body 120 and connector parts 122 and 124 at opposite ends. The connector parts have circular ratchet surfaces 126, 128 as shown with a hole 130 and an internally threaded hole 132. As will be seen, the ratchet surfaces 126, 128 are displaced at 90° relative to each other.

Referring to Figures 25 and 26, the bone fixing device adapted for use regarding pelvic bones of a patient, generally indicated by reference numeral 134, includes a central bar or link 136, two side links 138, 140 and two bone pin end carrying bars 142, 144 provided at the ends of the links 138, 140 respectively.

The links 138 and 140 are identical and the pin carrying bars 142 and 144 are identical.

The links 138, 140 are attachably connected to the central bar 136 by way of first connectors 146, which are identical, and the pin carrying bars 142, 144 carry bone pin holders 148, which are identical.

The identical components will only be described with reference to the components associated with the link 138 but the same description applies to the components associated with the link 140.

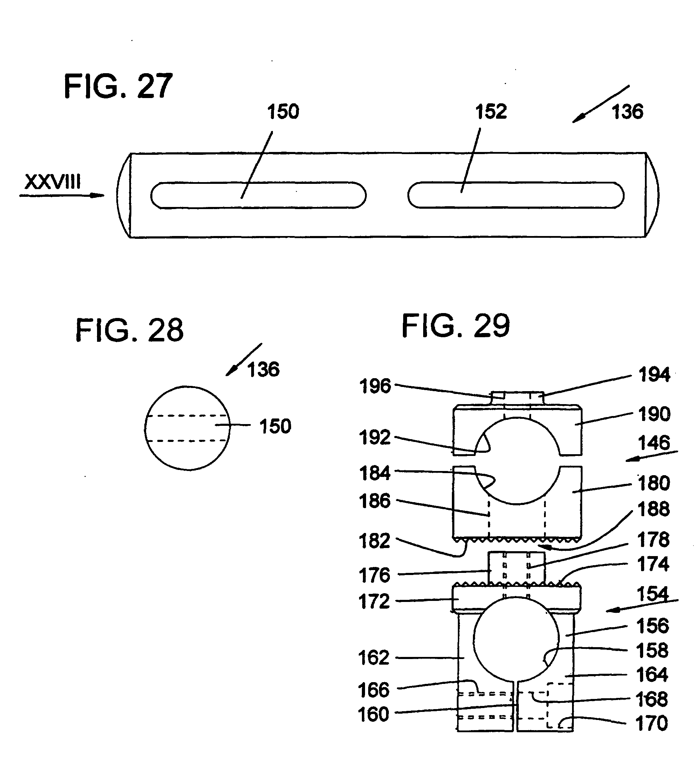

Referring to Figure 27, the central bar 136 is of elongated round shape and has two elongated slots 150, 152.

Referring to Figure 29, the connector 146 includes a base clamp 154 which has a body 156 which is split with a hole 158 and a gap 160 extending therefrom so as to define two body parts 162 and 164. The parts 162, 164 respectively have an internally threaded hole 166 and a hole 168 with a recess 170.

On top of the parts 162, 164 a disc shaped head 172 with a ratchet surface 174 and a boss 176 are located. The boss 176 has an internally threaded hole 178.

The connector 146 further has a central disc 180 having a bottom ratchet surface 182 and a semi-circular recess 184 opposite thereto. A hole 186 is provided in it extending from the recess 184 to the ratchet surface 176 and being open at 188 to receive the boss 176.

The connector 146 further has a top disc 190 having a semi-circular recess 192 and a boss 194 at its upper end in which a hole 196 passes down into the recess 192.

When pressed together the link 138 (or 140) is inserted through the hole 158 and is clamped thereto in any desired angular position by way of a threaded bolt (not shown) inserted into the hole 168 and screwed into the threaded hole 166.

The central part 180 is placed onto the part 156 in any desired position in that the boss 176 is placed into the hole 186. Thereafter the top disc 190 is placed onto the central disc 180 with the bar 136 fitting into the circular hole defined by the recesses 184 and 192. A pin (not shown) is passed through the hole 196 and through the slot 150 or 152 to be threaded into the threaded hole 178 for clamping the bar 136 tightly into position.

Thus by suitable adjustment of the connector 146 any desired angular position can be obtained between the bar 136 and the link 138 or link 140.

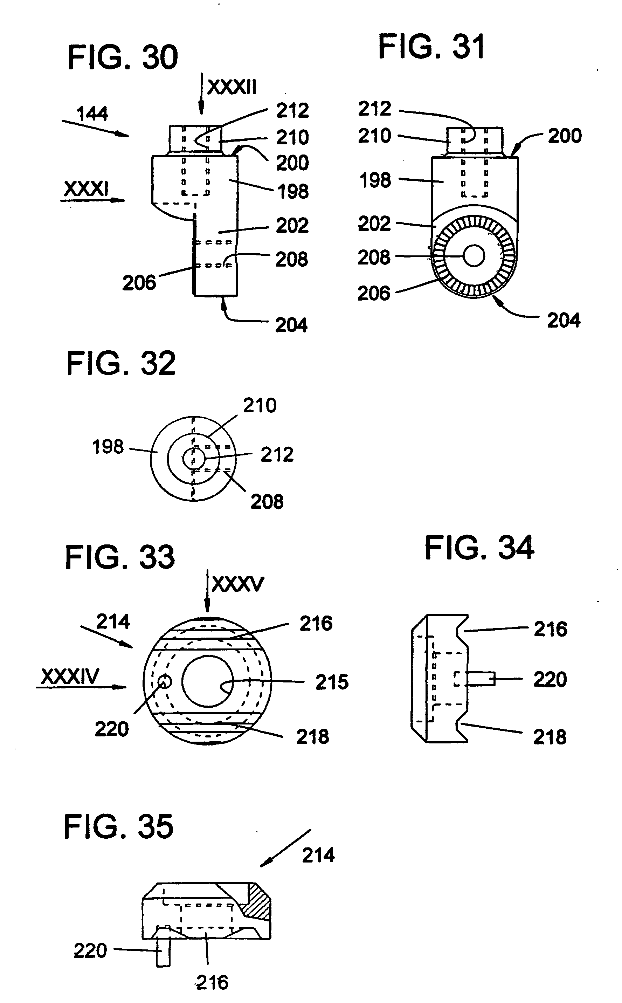

Referring now to Figures 30 to 38, the pin carrier bar 144 and its components are illustrated. The bar 144 has a round part 198 at its end 200 and a flat part 202 at its end 204. On the part 202 a round ratchet surface 206 is provided. Centrally an internally threaded hole 208 is provided. Although not shown, the link 138 at its end has a similar part with a round serrated formation. The ratchet surface 206 is placed into contact with this ratchet surface of the link 138 and is held in any desired angular position by way of a pin passing through the link 138 and being screwed into the threaded hole 208.

The part 198 has a stub 210 in which an intemally threaded hole 212 is provided.

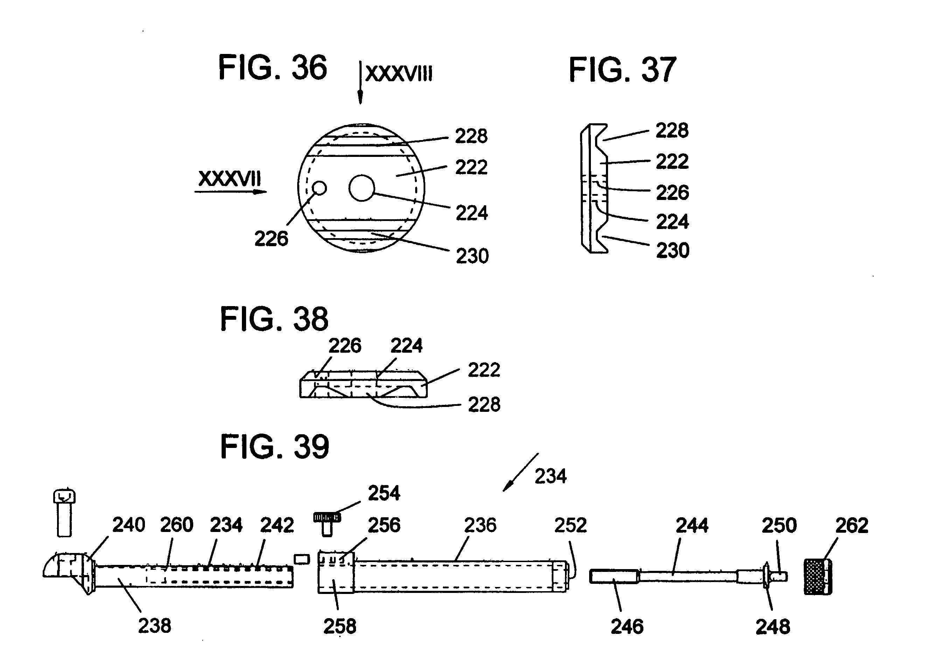

A pin carrier 148, illustrated in Figures 33 to 38 has a disc 214 with a central hole 215 for receiving the stub 210. It further has two grooves 216, 218 and a pin 220. These cooperate with a cap 222 illustrated in Figures 36 to 38. The cap 222 has a central hole 224 and a pin hole 226 to receive the pin 220.

The cap 222 further has two grooves 228, 230 which, together with the grooves 216 and 218 define clamping passages for the bone pins 232 shown in Figure 25.

A threaded screw (not shown) is passed through the hole 224 and is screwed into the threaded hole 212 for clamping the bone pins 232 in any desired position.

By means of the device 134 and its components as illustrated in Figures 25 to 38 it therefore is possible to adjust the various components in numerous directions and to provide a multitude of variations depending on the pelvic size of a patient and the particular position where the required bone pins are to be located. Once in position the bone pins are fixed exactly and are held securely in this position.

Figure 39 shows an exploded view of a further component to be used in conjunction with the bone fixing device as illustrated in the preceding drawings.

The device, generally indicated by reference numeral 234 is a compression release adjustment device. It includes a tubular body 236 into which a link 238 is insertable from one end. The link 238 has a head 240 with a serrated connector as explained above for connection to a complementary link. The link 238 further has an internally threaded hole 242. A bar 244 having a threaded end 246 and a collar 248 and square shaped head 250 at the opposite end is inserted into the tubular body 236 until the collar 248 abuts against its externally threaded end 252. The head 250 is turned so that the screw thread end 246 turns into the threaded link 238. A pin 254 having a threaded shank is received in a threaded hole 256 of a saddle 258 provided on the body 236 and the pin 254 slides in a slot 260 provided externally in the link 238 to prevent rotation thereof. By suitable rotation of the head 250 the length of the device 234 can be varied, and thereby the distance between any pin holders 148 fitted thereon. An end cap 262 is screwed onto the threaded end 252 to keep the bar 244 in position.

### List of references

- 10: bone fixing device
- 12: first link
- 14: second link
- 16: rounded end
- 18: connector part
- 20: internally threaded hole
- 22: ratchet surface
- 24: rounded end
- 26: connecting part
- 28: hole
- 30: ratchet surface
- 32: recess
- 34: bolt
- 36: head
- 38: bone pin holder
- 40: body
- 42: base disc
- 44: upper disc
- 46: body part
- 48: body part
- 50: hole
- 52: passage
- 54: threaded hole
- 56: hole
- 58: recess
- 60: bolt
- 62: shank
- 64: head
- 66: disc shaped head
- 68: upper face
- 70: pillar or boss
- 72: threaded passage
- 74: ratchet surface
- 76: engagement surface
- 78: groove
- 80: groove
- 82: pin
- 84: hole
- 86: bottom surface
- 88: recess
- 90: recess
- 92: hole
- 94: bone pin
- 96: bolt
- 98: shank
- 99: head
- 100: hole
- 102: link
- 104: connector part
- 106: connector part
- 108: cylindrical body
- 110: ratchet surface
- 112: ratchet surface
- 114: hole
- 116: hole
- 118: link
- 120: body
- 122: connector part
- 124: connector part
- 126: ratchet surface
- 128: ratchet surface
- 130: hole
- 132: hole
- 134: bone fixing device
- 136: link
- 138: link
- 140: link
- 142: bar
- 144: bar
- 146: connector
- 148: pin holder
- 150: slot
- 152: slot
- 154: base clamp
- 156: body
- 158: hole
- 160: gap
- 162: body part
- 164: body part
- 166: hole
- 168: hole
- 170: recess
- 172: head
- 174: ratchet surface
- 176: boss
- 178: threaded hole
- 180: central disc
- 182: ratchet surface
- 184: semi-circular recess
- 186: hole
- 188: opening
- 190: disc
- 192: recess
- 194: boss
- 196: hole
- 198: round part
- 200: end of bar
- 202: flat part
- 204: end of bar
- 206: ratchet surface
- 208: threaded hole
- 210: stub
- 212: threaded hole
- 214: disc
- 215: central hole
- 216: groove
- 218: groove
- 220: pin
- 222: cap
- 224: hole
- 226: pin hole
- 228: groove
- 230: groove
- 232: bone pin
- 234: bone fixing device
- 236: tubular body
- 238: link
- 240: head
- 242: hole
- 244: bar
- 246: threaded end
- 248: collar
- 250: head
- 252: threaded end
- 254: pin
- 256: threaded hole
- 258: saddle
- 260: slot
- 262: end cap

## Claims

1. A bone fixing device, which includes at least one elongated link (12, 14), and a number of clamp units (38) movably mounted on one such elongated link (12, 14), each elongated link (12, 14) being substantially round in end view, and each clamp unit (38) including a clamp body (40) slidably and rotatably mounted on its link (12, 14), with clamp locking means (60, 54, 56) being provided for locking the clamp body (40) on the link (12, 14), the clamp body further rotatably carrying a bone pin carrier (42, 44), which is adapted to receive at least one bone pin (94), the bone pin carrier (42, 44) including a base disc (42) rotatably mounted on the clamp body (40), and a top disc (44), carrier locking means (96) being provided for locking the bone pin carrier (42, 44) relative to the clamp body (40), the carrier locking means (96) being adapted to pull the top disc (44) and the base disc (42) relatively together to clamp at least one bone pin (94) between them; the top disc (44) and the base disc (42) have cooperating locating grooves (88, 90; 78, 80) for receiving bone pins (94); **characterized in that** a pin (82) is provided in the base disc (42) extending towards the top disc (44) and a hole (92) is provided in the top disc (44) for receiving the pin (82) so as to prevent relative rotation between the base disc (42) and the top disc (44) for aligning the locating grooves (88, 90; 78, 80) of the top disc (44) and the base disc (42) for receiving and locating the bone pin(s) (94).

2. A device as claimed in claim 1, characterized thereby that link connecting means (18) are provided for connecting a number of elongated links in series, the link connecting means including a projection (26) at an end of an elongated link (14), and a locking surface (30) is provided on the projection (26) adapted to be locked to a similar locking surface (22) provided on a projection (18) of another elongated link (12).

3. A device as claimed in claim 2, characterized thereby that the locking surface is a ratchet surface (22, 30).

4. A device as claimed in any one of the preceding claims, characterized thereby that the carrier locking means (96) includes a threaded bolt (96), adapted to pass through a hole (100) in the top disc (44) and a hole (84) in the base disc (42) to be threaded into a threaded hole (72) in the clamp body (40).

5. A device as claimed in any one of the preceding claims, characterized thereby that a connecting link (136) with at least one groove (150, 152) is provided, and link connectors (146) for connecting the connecting link (136) to two elongated links (138, 140).

6. A device as claimed in any one of the preceding claims, characterized thereby that at least one of the elongated links (234) includes an outer sleeve (236) and an inner rod (238) extending out of the outer sleeve (236) and being connected to another elongated link (12, 14), and adjustment means (244, 262, 260) for adjusting the inner rod (238) relative to the outer sleeve (236).

## Patentansprüche

1. Knochen-Fixierungsvorrichtung mit mindestens einem länglichen Verbindungsglied (12,14) und einer Anzahl von Klemmeinheiten (38), die auf einem derartigen länglichen Verbindungsglied (12,14) beweglich montiert sind, wobei jedes längliche Verbindungsglied (12,14) in Stirnseitenansicht im wesentlichen rund ist und jede Klemmeinheit (38) einen Klemmkörper (40) aufweist, der verschiebbar und drehbar auf seinem Verbindungsglied (12,14) montiert ist, wobei Klemmen-Schließmittel (60,54,56) zum Schließen des Klemmkörpers (40) um das Verbindungsglied (12,14) vorgesehen sind, wobei der Klemmkörper ferner drehbar einen Knochen-Bolzenträger (42,44) trägt, der dazu ausgelegt ist, mindestens einen Knochenbolzen (94) aufzunehmen, wobei der Knochen-Bolzenträger (42,44) eine Grundscheibe (42), die drehbar auf dem Klemmkörper (40) montiert ist, und eine obere Scheibe (44) aufweist, wobei ein Träger-Feststellmittel (96) vorgesehen ist, um den Knochen-Bolzenträger (42,44) in bezug auf den Klemmkörper (40) festzustellen, wobei das Träger-Feststellmittel (96) dazu ausgelegt ist, die obere Scheibe (44) und die Grundscheibe (42) aufeinander zu zu ziehen, um mindestens einen Knochenbolzen (94) zwischen ihnen einzuklemmen; wobei die obere Scheibe (44) und die Grundscheibe (42) miteinander zusammenwirkende Fixiernuten (88,90;78,80) zur Aufnahme von Knochenbolzen (94) aufweisen;
**dadurch gekennzeichnet, daß** ein Bolzen (82) in der Grundscheibe (42) und sich zur oberen Scheibe (44) erstreckend vorgesehen ist und ein Loch (92) in der obere Scheibe (44) zur Aufnahme des Bolzens (82) vorgesehen ist, um die relative Drehung zwischen der Grundscheibe (42) und der oberen Scheibe (44) zu vermeiden, um die Fixiernuten (88,90; 78,80) der oberen Scheibe (44) und der Grundscheibe (42) zur Aufnahme und Fixierung des Knochenbolzens/der Knochenbolzen (94) auszurichten.

2. Vorrichtung nach Anspruch 1, **dadurch gekennzeichnet, daß** Verbindungsglied-Anschlußmittel (18) vorgesehen sind, um eine Anzahl länglicher Verbindungsglieder in Reihe aneinander anzuschließen, wobei die Verbindungsglied-Anschlußmittel einen Vorsprung (26) an einem Ende eines länglichen Verbindungsgliedes (14) aufweisen und eine Eingriffsfläche (30) an dem Vorsprung (26) vorgesehen ist, welche dazu ausgelegt ist, mit einer ähnlichen Eingriffsfläche (22) an einem Vorsprung (18) eines anderen länglichen Verbindungsgliedes (12) in Eingriff zu kommen.

3. Vorrichtung nach Anspruch 2, **dadurch gekennzeichnet, daß** die Eingriffsfläche eine Ratschenfläche (22,30) ist.

4. Vorrichtung nach einem der vorangehenden Ansprüche, **dadurch gekennzeichnet, daß** das Träger-Feststellmittel (96) einen Gewindebolzen (96) aufweist, der dazu ausgelegt ist, durch ein Loch (100) in der oberen Scheibe (44) und ein Loch (84) in der Grundscheibe (42) hindurch zu ragen, um in eine Gewindebohrung (72) in dem Klemmkörper (40) eingeschraubt zu werden.

5. Vorrichtung nach einem der vorangehenden Ansprüche, **dadurch gekennzeichnet, daß** ein Verbindungsglied (136) mit mindestens einer Nut (150,152) vorgesehen ist und daß Verbindungsglied-Anschlußelemente (146) zum Anschließen des Verbindungsgliedes (136) an zwei längliche Verbindungsglieder (138,140) vorgesehen sind.

6. Vorrichtung nach einem der vorangehenden Ansprüche, **dadurch gekennzeichnet, daß** mindestens eines der länglichen Verbindungsglieder (234) eine äußere Buchse (236) und einen inneren Stab (238) aufweist, der sich aus der äußeren Hülse (236) heraus erstreckt und an ein anderes längliches Verbindungsglied (12,14) angeschlossen ist, und ferner Einstellmittel (244, 262,260) aufweist zum Einstellen des inneren Stabs (238) in bezug auf die äußere Buchse (236).

## Revendications

1. Dispositif de fixation d'os comprenant au moins une tige d'assemblage oblongue (12, 14), et une pluralité d'unités de fixation (38) montées déplaçables sur ladite tige d'assemblage oblongue (12, 14), chaque tige d'assemblage oblongue (12, 14) étant sensiblement de forme cylindrique dans une vue en bout, et chaque unité de fixation (38) comprenant un corps de fixation (40) monté en rotation et en translation sur sa tige d'assemblage (12, 14), avec des moyens de blocage du corps de fixation (60, 54, 56) agencés pour bloquer le corps de fixation (40) sur la tige d'assemblage (12, 14), le corps de fixation supportant en outre de façon pivotante un porte broche à os (42, 44), qui est adapté pour recevoir au moins une broche à os (94), le porte broche à os (42, 44) comprenant un disque d'embase (42) monté pivotant sur le corps de fixation (40), et un disque de sommet (44), des moyens de blocage du porte broche (96) étant agencés pour bloquer le porte broche à os (42, 44) par rapport au corps de fixation (40), les moyens de blocage du porte broche (96) étant adaptés pour tirer le disque de sommet (44) et le disque d'embase (42) l'un par rapport à l'autre pour pincer entre eux au moins une broche à os (94), le disque de sommet (44) et le disque d'embase (42) ayant des rainures de positionnement coopérantes (88, 90; 78, 80) pour recevoir les broches à os (94), **caractérisé en ce qu'**un ergot (82) est agencé sur le disque d'embase (42) en s'étendant vers le disque de sommet (44) et qu'un orifice (92) est réalisé dans le disque de sommet (44) pour recevoir l'ergot (82) de façon à empêcher une rotation relative entre le disque d'embase (42) et le disque de sommet (44) pour obtenir l'alignement des rainures de positionnement (88, 90; 78, 80) du disque de sommet (44) et du disque d'embase (42) pour recevoir et positionner la (les) broche(s) à os (94).

2. Un dispositif selon la revendication 1, **caractérisé en ce que** des moyens de connexion de tiges d'assemblage (18) sont prévus pour connecter en série une pluralité de tiges d'assemblage oblongues, les moyens de connexion de tiges d'assemblage comprenant une partie en saillie (26) à une extrémité d'une tige d'assemblage oblongue (14), et qu'une surface de blocage (30) est agencée sur la partie en saillie (26) adaptée pour être bloquée sur une surface de blocage similaire (22) agencée sur une partie en saillie d'une autre tige d'assemblage oblongue (12).

3. Un dispositif selon la revendication 2, **caractérisé en ce que** la surface de blocage est une surface à rochets (22, 30).

4. Un dispositif selon l'une quelconque des revendications précédentes, **caractérisé en ce que** les moyens de blocage du porte broche (96) comprennent un boulon fileté (96) adapté pour passer par un orifice (100) dans le disque de sommet (44) et par un orifice (84) dans le disque d'embase (42) pour être vissé dans un orifice taraudé (72) dans le corps de fixation (40).

5. Un dispositif selon l'une quelconque des revendications précédentes, **caractérisé en ce que** sont prévus une tige de liaison (136) avec au moins une rainure (150, 152), et des connecteurs de tiges (146) pour relier la tige de liaison (136) à deux tiges d'assemblage oblongues (138, 140).

6. Un dispositif selon l'une quelconque des revendications précédentes, **caractérisé en ce qu'**au moins l'une des tiges d'assemblage oblongues (234) comprend un manchon externe (236) et une barre intérieure (238) s'étendant à l'extérieur du manchon externe (236) et étant reliée à une autre tige d'assemblage oblongue (12, 14), et des moyens d'ajustement (244, 262, 260) pour ajuster la barre intérieure par rapport au manchon externe (236).
